# EUROPEAN PATENT SPECIFICATION

(11) **EP 0 431 839 B1**
(45) Date of publication and mention of the grant of the patent: **09.08.1995**
(21) Application number: 90312990.6
(22) Date of filing: 29.11.1990
(51) Int. Cl.: C12N 15/00, C12N 15/87, C12N 5/10, A61K 35/52

(54) **Buffer solution**
Pufferlösung
Solution tampon

(30) Priority: 03.12.1989 IL 92529
(43) Date of publication of application: 12.06.1991
(73) Proprietor: SCOPUS-GENETICS (ISRAEL) LTD., Ramat-Gan, 52520 (IL)
(72) Inventor: Gruenbaum,Joseph, Mevasseret Zion (IL); Fainsod,Abraham, 93103 Jerusalem (IL); Revel,Elie, Jerusalem (IL); Yarus,Sinai, Jerusalem (IL)
(74) Representative: Bizley, Richard Edward

(56) References cited:
- WO-A-90/08192
- PROCEEDINGS OF THE NATIONAL ACADEMY OF SCIENCES OF THE USA, vol. 68, no. 2, February 1971, pages 353-357; B.G. BRACKETT et al.: "Uptake of heterologous genome by mammalian spermatozoa and its transfer to ova through fertilization"

## Description

The present invention relates to buffer solutions, particularly suitable for preserving sperm cells. The same buffer solutions inter alia allow for the efficient longterm storage of sperm cells. The present invention also relates to manufacture of the said buffer solutions.

In the art, FM medium (solution) was developed by Lavitrano et al. (Lavitrano, M., et al., Cell 57, 717 - 723 (1989)) for maintaining sperm cells in in vitro fertilization experiments. The medium was based on Whittingham's Tyrode solution (Whittingham, D.G., J. Reprod. Fert. Suppl. 14, 7-21 (1971)), from which sodium lactate, penicillin, and streptomycin were omitted; NaH₂PO₄ was replaced by 0.15 mM Na₂HPO₄ and NaCl was increased to 120 mM. The medium was supplemented with 30 mg/ml bovine serum albumin.

The present invention provides novel buffer solutions (hereafter referred to as ERS buffer) with advantages over previously disclosed buffers in the maintenance of sperm cells. These advantages include increased viability of sperm cells transiently maintained or stored for long periods of time in ERS buffer, and increased efficiency of fertilization by artificial insemination using sperm cells maintained in said buffer compared to similar cells maintained in, for example, FM solution (Lavitrano, M., et al. supra).

Thus, the invention provides a novel aqueous buffer solution containing from 87 mM to 174 mM NaCl, from 17.4 mM to 34.8 mM KCl, from 3.3 mM to 34.8 mM CaCl₂, from 1.5 mM to 3.1 mM MgSO₄ and from 21 mM to 44 mM NaHCO₃, having a pH of about 7.2 and optionally further comprising conventional additives, optionally said buffer solution containing about 116 mM NaCl, about 23 mM KCl, about 6 mM CaCl₂, about 2 mM MgSO₄ and about 29 mM NaHCO₃.

In the accompanying drawings:-
- Figure 1: shows the viability of rooster sperm cells at 4°C, room temperature (RT, app. 22°C) and 37°C in ERS buffer compared to that in seminal fluid, as judged by the motility of the sperm.
- Figure 2A: shows the effect of different glycerol concentrations on the ability to use the ERS buffer solution to freeze rooster sperm cells. The viability of sperm cells was judged by the percent of motile sperm.
- Figure 2B: shows the effect of ampicillin, streptomycin and kanamycin on the ability to use ERS buffer solution to store rooster sperm cells. The viability of sperm cells was judged by the percent of motile sperm.
- Figure 3: shows the viability of ostrich sperm cells at 4°C, room temperature, following freezing in 10% glycerol and 15% glycerol diluted 1:1, 1:4 and 1:10 in ERS buffer solution. Sperm cells that were stored in the seminal fluid were practically dead following 5 hrs of incubation either at 4°C or at room temperature.

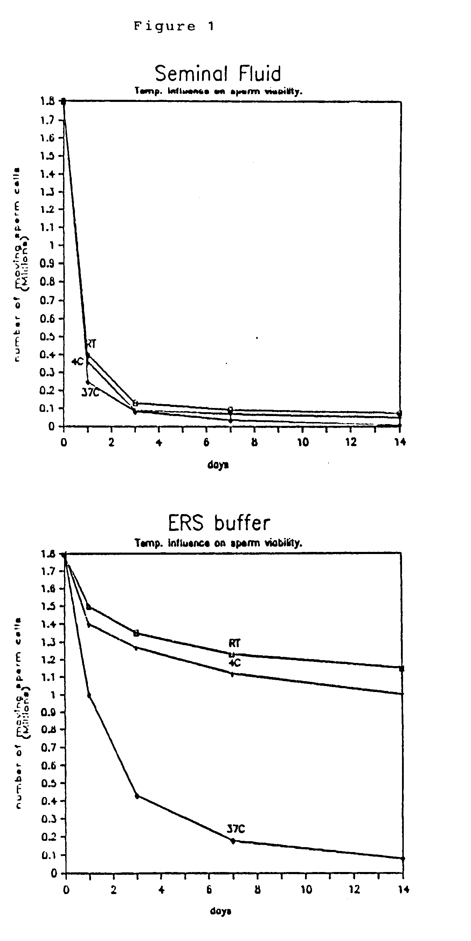

A buffered solution has been developed that maintains and prolongs viability of sperm. The inventors have found that a buffer solution containing from 87 mM to 174 mM NaCl, from 17.4 mM to 34.8 mM KCl, from 3.3 mM to 34.8 mM CaCl₂, from 1.5 mM to 3.1 mM MgSO₄ and from 21 mM to 44 mM MaHCO₃, having a pH of about 7.2 fulfils the said requirement very efficiently. Preferably, a buffer solution containing about 116 mM MaCl, about 23 mM KCl, about 6 mM CaCl₂, about 2 mM MgSO₄ and about 29 mM NaHCO₃, having a pH of about 7.2, is used. Preparation of the ERS buffer will be described in Example 1. The viability of, for example, rooster sperm cells diluted with the ERS buffer solution is shown hereafter in Example 2 and in Fig. 1. The invention therefore relates to the said novel ERS buffer solution. Furthermore, conventional cryo-preserving agents such as, e.g. glycerol, may be added to the ERS buffer for prolonged preservation under deep-freezing. Additionally, the present buffer solution may optionally contain antibiotics, such as ampicillin, streptomycin and kanamycin. As will be shown in the following examples, the addition of antibiotics has no negative effect on the viability of sperm cells and the addition of antibiotics is particularly recommended to avoid possible bacterial contamination during storage.

Sperm cells can be collected from an animal by conventional methods and can be diluted with the said ERS buffer solution to a desired concentration. At this stage the sperm may be further used immediately or it may be stored either at room temperature or at a lower temperature, e.g. 4°C. Under these conditions, as already referred to above, and described in Example 1 and Fig. 1, viability is well preserved. Storage in liquid N₂ is also possible, preferably with the addition of cryo-preservants such as glycerol. Storage of rooster sperm cells diluted in ERS buffer containing about 15% glycerol resulted in over 95% viability of the cells, following thawing at 4°C. The use of the ERS buffer of the invention in sperm cell storage is, thus, an important aspect of the invention.

With the use of the ERS buffer of the invention, the efficiency of artificial insemination of birds, preferably domesticated birds, increases compared to undiluted sperm or sperm diluted 1:10 with FM solution (Lavitrano, M., et al. supra). Using, for example, White Leghorn hens, the success of artificial insemination using the ERS buffer was 4 times greater, compared to the FM medium, and 20% higher, compared to the fresh, undiluted sperm.

The invention will now be described in more detail on hand of the following examples. Some of the following examples specifically refer to experiments performed with White Leghorn hens. It should be noted that all experiments performed with said strain were repeated with additional lines of the White Leghorn hens and with White Plymouth Rock hens, with substantially the same results.

The following examples are illustrative only and do not in any sense limit the invention, which is only defined by the scope of the appended claims.

### EXAMPLES

### Example 1

### Preparation of ERS buffer

An aqueous solution containing about 116 mM NaCl, about 23 mM KCl and about 6 mM CaCl₂ is prepared by dissolving said components in water. MgSO₄, to a final concentration of about 2 mM, and NaHCO₃ to a final concentration of about 29 mM, are then added to the solution. pH is about 7.2. The solution is then filtered through a 0.22»m filter. The filtered solution consitutes the ERS buffer according to the invention.

Conventional cryo-preservants such as glycerol may be added to the ERS buffer for long-term cryo-preservation of the diluted sperm. For example, glycerol may be added at a concentration of about 15%, giving a buffer suitable for preservation of the sperm in liquid N₂.

### Example 2

### (1) Viability of sperm in ERS buffer

### (A)(i) White Leghorn rooster sperm

Immediately after obtaining the sperm from the rooster, by conventional methods, the sperm is diluted about 1:10 in the ERS buffer. Unless the sperm, is immediately used, it can be stored at 4°C or at room temperature. Storing the sperm cells in this buffer at 4°C or at room temperature for two weeks resulted in over 80% viability of the sperm as judged from microscopic analysis of the number of motile sperm cells in the sample (Fig. 1). Addition of about 15% glycerol to the ERS buffer enables the storage of the sperm cells in liquid N₂, with over 95% sperm viability when thawed at 4°C.

Further experiments were conducted to establish the efficiency of fertilization with rooster sperm diluted in the ERS buffer.

Sperm was diluted as follows:
- First group:: Undiluted sperm. Artificial insemination of 40 hens (A).
- Second group:: Sperm diluted 1:10 in the ERS buffer. Artificial insemination of 80 hens (D).

Each time, sperm was obtained from a whole group of roosters (about 10 individuals), immediately diluted to the desired concentration and immediately used for artificial insemination, each hen inseminated with 0.1 ml sperm solution. Eggs were collected and incubated in a Victoria incubator, under shaking at 1 rph, under optimal temperature and humidity conditions.

Following incubation for 24-72 hrs., the eggs were opened and the percentage of fertilized eggs in each batch checked.

Results were as follows:

| **Days 2 and 3 post artificial insemination:** | | |
|---|---|---|
| Group | A | D |
| No. of eggs (total) | 56 | 127 |
| Unfertilized eggs | 3 | 8 |
| % fertilized eggs No. of eggs (total) | 94.6 58 | 93.7 123 |

| **Days 5 and 6 post artificial insemination** | | |
|---|---|---|
| No. of eggs (total) | 58 | 123 |
| Unfertilized eggs | 3 | 14 |
| % fertilized eggs No. of eggs (total) | 94.8 114 | 88.6 250 |

| **Days 2,3,5,6 post insemination - Inclusive results:** | | |
|---|---|---|
| No. of eggs (total) | 114 | 250 |
| Unfertilized eggs | 6 | 22 |
| % fertilized eggs | 94.7 | 91.2 |

The slight decrease in the percentage of fertilization in days 5 and 6, using diluted sperm, is mainly due to eggs laid on day 6 post insemination. There is no control for % fertilized eggs of hens from group D inseminated with undiluted sperm.

### (A)(ii) Ostrich

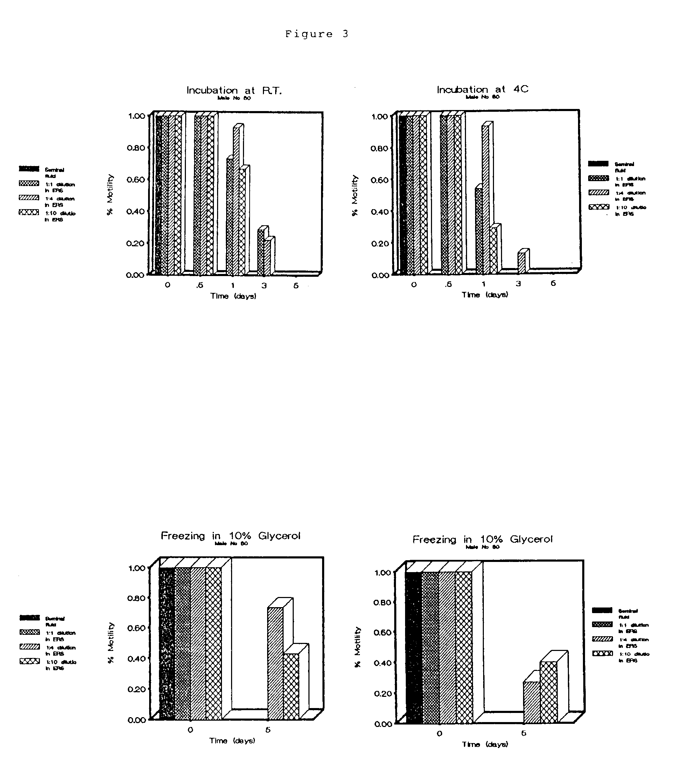

Ostrich sperm was collected in the Ostrich Farm of Kibbutz HaOn, Israel, from male No. 80. The sperm cell density was measured and found to be 5x10⁹ sperm cells/ml. The sperm cells were immediately diluted in the ERS buffer 1:10, 1:4 and 1:1 in final volumes between 0.3 ml to 1 ml. Undiluted sperm cells were used as controls. Following 1 hr incubation at room temperature, 4 samples from each dilution were stored on ice and the other two samples were left at room temperature. After additional 4 hrs of incubation, glycerol was added into two of the samples that were stored on ice, to give final concentrations of 10% and 15% respectively, and these samples were stored in liquid nitrogen. As shown in Figure 3, following 24 hrs of incubation the viability of the ostrich sperm cells that were stored at room temperature, in all dilutions, was over 65%, as judged by their motility. After 48 hrs of incubation, over 20% of the sperm cells in the dilutions of 1:1 and 1:4 were still viable. Following freezing in liquid nitrogen, up to 75% of the sperm cells were still viable. The best results were obtained when the concentration of glycerol was 10% and the dilution in ERS buffer was 1:4.

### (A)(iii) Geese

Gender sperm was collected from genders in the farm of Kibbutz Kiryat Anavim, Israel, and diluted with ERS buffer as follows:

| Gender No. | Vol. of sample ml | Sperm Cell Density cells/ml | Final Vol. ml |
|---|---|---|---|
| 2002 | 0.3 | <10⁴ | 3.0 |
| 2032 | 0.5 | 1.3x10⁸ | 5.0 |
| 1974 | 0.3 | 10⁸ | 3.0 |
| 1322 | 1.0 | 5x10⁷ | 10.0 |
| 1620 | nr* | 10⁷ | nr* |

| | | | |
|---|---|---|---|
| *nr - not recorded | | | |

Following dilution with the buffer, an aliquot of each sample was kept at room temperature, another aliquot at 4°C (ice bucket). The aliquots were microscopically observed, there are no data for zero time.

After 5 hours of incubation, good motility of over 95% was observed in all of the samples, both those maintained at room temperature and those kept at 4°C. The sperm appeared morphologically intact. Glycerol to a final concentration of 10% was added to the aliquots kept at 4°C, and these aliquots were frozen in liquid nitrogen and stored at -80°C.

After 24 hours of incubation, the aliquots maintained at room temperature were bacterially contaminated, therefore it was difficult to assess the capability of the buffer of preserving the sperm. Still, even under these conditions, 50% motility in the sperm of No. 2032 and 25% motility in the sperm of Nos. 1974, 1322 and 1620 was observed. Motility was weaker than that observed after 5 hours from dilution.The deep-frozen samples of Nos. 2032 and 1974 were thawed after 4 days of storage at -80°C. Sperm motility exceeded 60%.

It has thus been shown that the ERS buffer may be efficiently used for diluting and preserving gender sperm, for time periods of at least 4 hours, preservation is possible at both room temperature and 4°C and the sperm can be efficiently preserved also under deep freezing conditions, when diluted in the ERS buffer.

### (B) Outbred Sabra mouse sperm

Outbred Sabra mouse sperm cells were obtained from the mouse by dissecting out the epididymis of the mouse. The sperm cells were transferred into FM (7) or ERS buffers. Following six hours incubation at 4°C or at room temperature, over 90% viability of the sperm cells was observed in both buffers. In addition, following the six hours incubation, practically no agglutination of the cells was observed when the sperm cells were incubated in the ERS buffer, while in the FM buffer a large fraction of the sperm cells agglutinated.

### (2) Effect of different concentrations of glycerol on sperm viability following freezing

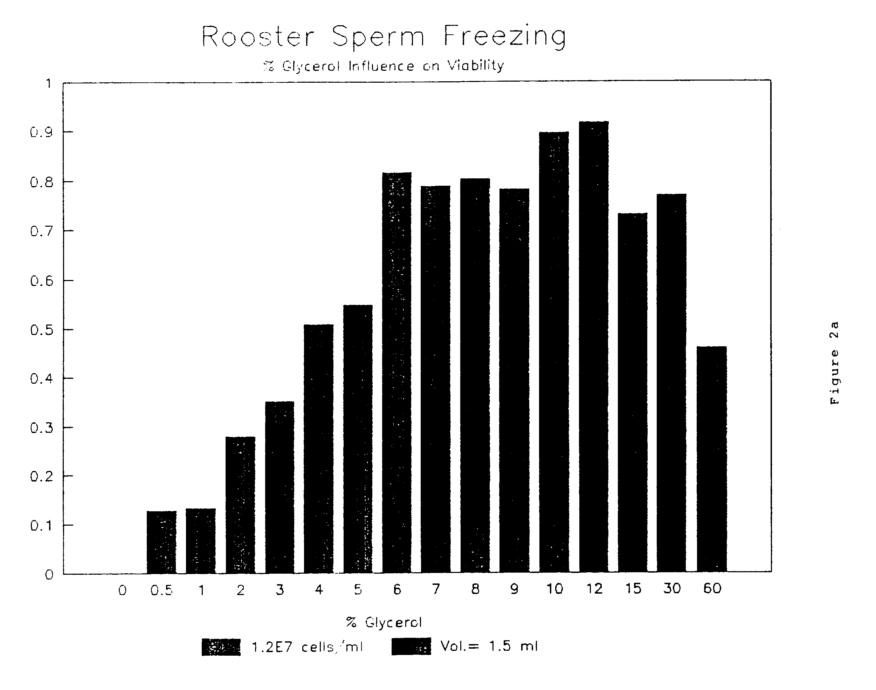

The effect of different glycerol concentrations on sperm cell viability following freezing of the sperm cells in liquid nitrogen is shown in Figure 2(A). The sperm was diluted 1:10 in ERS buffer and glycerol was added to the desired concentration in a final volume of 1.5 ml. Viable sperm cells were observed in concentrations between 0.5% and 60%. Over 90% viability was obtained in concentrations between 6% and 12% of glycerol.

Frozen sperm cells can be used for artificial insemination. Frozen sperm cells were thawed several days or weeks after their freezing in liquid nitrogen and used to artificially inseminate chickens. Eggs were collected in the same day they were laid and incubated. Viable chickens hatched from these eggs.

### (3) Effect of antibiotics

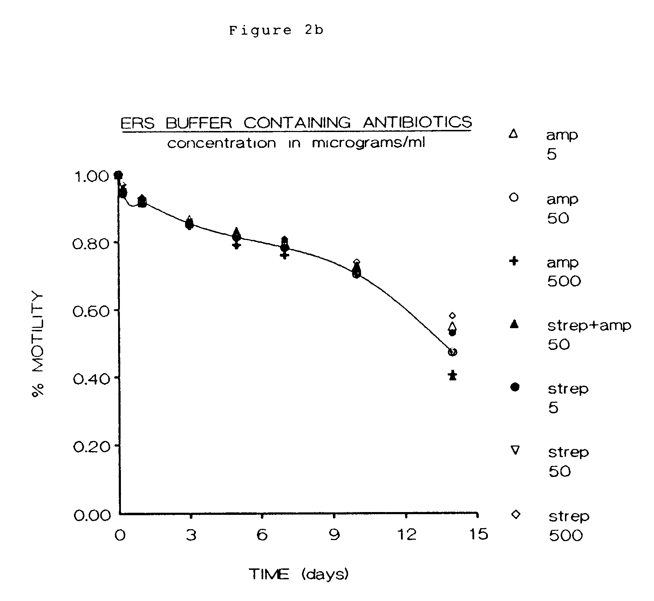

The effect of three antibiotic substances, ampicillin, streptomycin and kanamycin, and a combination of ampicillin and streptomycin in the ERS buffer, in concentrations of up to 500 »g/ml, on the viability of the sperm cells was analyzed. No negative effect of these antibiotics on the rooster sperm cells viability was observed. Results are shown in Fig. 2(B). Thus, to avoid possible bacterial contamination in the course of storage of sperm cells, the addition of, for example, up to 50 »g/ml of ampicillin and streptomycin is recommended.

### Example 3

### Efficiency of artificial insemination by the sperm cells

With the use of the ERS buffer the efficiency of artificial insemination increases compared to undiluted sperm or sperm diluted 1:10 with FM solution (Lavitrano, M., et al. supra). Using White Leghorn hens, the success of artificial insemination using the ERS medium was 4 times higher compared to the FM medium and 20% higher compared to the fresh undiluted sperm.

## Claims

1. An aqueous buffer solution containing from 87 mM to 174 mM NaCl, from 17.4 mM to 34.8 mM KCl, from 3.3 mM to 34.8 mM CaCl₂, from 1.5 mM to 3.1 mM MgSO₄ and from 21 mM to 44 mM NaHCO₃, having a pH of about 7.2 and optionally further comprising conventional additives, optionally said buffer solution containing about 116 mM NaCl, about 23 mM KCl, about 6 mM CaCl₂, about 2 mM MgSO₄ and about 29 mM NaHCO₃.

2. A buffer solution according to claim 1 further comprising a preserving agent, optionally glycerol, at a concentration of from 0.5 to 60%, especially from 6 to 30%, and/or the buffer solution further containing an antibiotic substance, optionally ampicillin, streptomycin or kanamycin or mixtures thereof.

3. A method of manufacture of a buffer solution as defined in claim 1 comprising adding the MgSO₄ and MaHCO₃ to the other components in solution at the final dilution and filtering the resulting solution through a 0.22 »m filter.

4. The use of a buffer as defined in claim 1 or claim 2 in storage of sperm cells.

## Patentansprüche

1. Eine wässrige Pufferlösung enthaltend 87 mM bis 174 mM NaCl, 17.4 mM bis 34.8 mM KCl, 3.3 mM bis 34.8 mM CaCl₂, 1.5 mM bis 3.1 mM MgSO₄ und 21 mM bis 44 mM NaHCO₃ mit einem pH-Wert von etwa 7.2 und gegebenenfalls weitere übliche Additive, wobei die Pufferlösung gegebenenfalls etwa 116 mM NaCl, etwa 23 mM KCl, etwa 6 mM CaCl₂, etwa 2 mM MgSO₄ und etwa 29 mM NaHCO₃ enthält.

2. Eine Pufferlösung gemäß Anspruch 1 umfassend weiterhin ein Konservierungsmittel, gegebenenfalls Glycerin, in einer Konzentration von 0.5 bis 60%, vorzugsweise von 6 bis 30%, und/oder ein Antibiotikum umfassend, gegebenenfalls Ampicillin, Streptomycin oder Kanamycin oder deren Gemische.

3. Verfahren zur Herstellung einer Pufferlösung gemäß Anspruch 1, umfassend das Zusetzen des MgSO₄ und des NaHCO₃ zu den anderen Komponenten in Lösung bei der Endverdünnung und Filtrieren der entstandenen Lösung durch einen 0.22 »m Filter.

4. Die Verwendung eines Puffers gemäß Anspruch 1 oder 2 zum Aufbewahren von Samenzellen.

## Revendications

1. Solution aqueuse tampon contenant du NaCl de 87 mM à 174 mM, du KCl de 17,4 mM à 34,8 mM, du CaCl₂ de 3,3 mM à 34,8 mM, du MgSO₄ de 1,5 mM à 3,1 mM et du NaHCO₃ de 21 mM à 44 mM, présentant un pH d'environ 7,2 et comprenant éventuellement d'autres additifs conventionnels, ladite solution tampon contenant éventuellement du NaCl environ 116 mM, du KCl environ 23 mM, du CaCl₂ environ 6 mM, du MgSO₄ environ 2 mM et du NaHCO₃ environ 29 mM.

2. Solution tampon selon la revendication 1, comprenant en outre un agent conservateur, éventuellement du glycérol à une concentration comprise entre 0,5 et 60%, plus particulièrement entre 6 et 30 %, et/ou ladite solution tampon contenant en outre une substance à activité antibiotique, éventuellement de l'ampicilline, de la streptomycine ou de la kanamycine ou des mélanges de ces substances.

3. Procédé de préparation d'une solution tampon selon la revendication 1, comprenant l'addition de MgSO₄ et NaHCO₃ aux autres constituants de la solution dans sa dilution finale et la filtration de la solution résultante au travers d'un filtre de 0,22 »m.

4. Utilisation d'un tampon selon la revendication 1 ou 2 pour la conservation de cellules spermatiques.
